# EUROPÄISCHE PATENTANMELDUNG

(11) **EP 0 891 754 A2**
(43) Veröffentlichungstag der Anmeldung: **20.01.1999**
(21) Anmeldenummer: 98890212.8
(22) Anmeldetag: 17.07.1998
(51) Int. Cl.: A61F 2/30, A61L 27/00

(54) **Implantat, insbesondere Gelenk-Prothesenimplantat**

(30) Priorität: 18.07.1997 AT 1232/97
(71) Anmelder: Implantech Medizintechnik Ges.m.b.H., 2344 Maria Enzersdorf (AT)
(72) Erfinder: Wurzinger, Anton, Dr., 2344 Maria Enzersdorf (AT)
(74) Vertreter: Kopecky, Helmut, Dipl.-Ing. Kopecky & Schwarz Patentanwälte

(57) **Zusammenfassung**

Bei einem Implantat mit einer strukturierten Oberfläche an den mit einem Knochen in Kontakt gelangenden Flächen ist zwecks Erzielung einer gezielten Porosität die strukturierte Oberfläche wie folgt gestaltet:

Die strukturierte Oberfläche ist aus vollem Material gebildet und mit einer Vielzahl zylindrischer Vertiefungen (1), insbesondere Bohrungen, mit Durchmessern von 0,3 bis 1 mm, vorzugsweise 0,5 bis 1 mm, und mit Tiefen zwischen 0,3 und 1 mm, vorzugsweise 0,5 und 1 mm, versehen, so daß eine Porosität zwischen 50 und 80 %, vorzugsweise 60 und 70 %, bewirkt ist.

## Beschreibung

Die Erfindung betrifft ein Implantat, insbesondere Gelenk-Prothesenimplantat, mit knochenseitigen strukturierten Oberflächen.

Vor allem bei einer zementlosen Implantation spielt die Osseointegration eine besondere Rolle, um die Stabilität des Implantates über einen längeren Zeitraum zu gewährleisten. Es ist daher erforderlich, daß die mit der Knochenstruktur kontaktierende Oberfläche Eigenschaften aufweist, die den Knochen anregen, durch Aufbau von neuen Knochenstrukturen an diese Oberfläche heran- und hineinzuwachsen. Da sich glatte Flächen als untauglich erwiesen haben, hat man bereits vorgeschlagen, die Oberfläche des Implantats an der Berührungsfläche mit der Knochenstruktur porös zu gestalten bzw. aufzurauhen, also gitterförmig, pyramidenförmig, kassettenförmig u. dgl. zu gestalten. Man hat auch bereits vorgeschlagen, auf diese Oberfläche Kugeln aufzusintern. Sinterprozesse können jedoch ungünstige Materialeigenschaften hervorrufen. Weiters besteht die Gefahr, daß sich Kugeln ablösen.

Es ist auch bereits bekannt, die Oberflächen der Implantate im Spritzgußverfahren herzustellen. Die so gebildeten Oberflächen weisen eine ausgezeichnete Aufrauhung auf. Nachteilig ist jedoch, daß sich die Porosität nicht exakt bestimmen läßt. Vielmehr sind die Erhöhungen und Vertiefungen willkürlich über die Oberfläche verteilt und nicht den Anforderungen angepaßt. Sind die Aufrauhungen zu flach, ist die Porosität für eine gute Osseointegration zu gering, sind die Aufrauhungen zu stark ausgeprägt, leidet die Festigkeit darunter. In diesem Fall stehen erhöhtes Porenvolumen und Festigkeit in einem ungünstigen Verhältnis zueinander. Weiters können gebirgsartige Vorsprünge abbrechen und es können die Verbindungsbrücken geschwächt werden. Dies alles führt dazu, daß die Oberfläche für eine gute Osseointegration nicht optimal ist.

Aus der US 5 489 306 A ist ein für die Einsetzung in den Hohlraum eines langen Knochens vorgesehenes Implantat bekannt, welches mehrere poröse Zonen mit unterschiedlicher Porengröße aufweist, derart, daß die Porengröße vom proximalen Bereich zum distalen Bereich abnimmt. Ein derartiges Implantat ist nur für den Einsatz in langen Knochen verwendbar.

Aus der EP 0 566 427 A2 ist es bekannt, bei einem Implantat eine strukturierte Oberfläche zu bilden, indem auf das Implantat eine Vielzahl von mit Ausnehmungen versehenen Folien bzw. dünnen Schichten aufgebracht und mit diesem und den Knochen nach Einsetzen in den Körper verbunden werden. Ein Implantat dieser Art erfordert einen hohen Herstellungsaufwand und ist auch schwierig beim Einsetzen handzuhaben.

Die Erfindung bezweckt die Vermeidung dieser Nachteile und Schwierigkeiten und stellt sich die Aufgabe, ein Implantat der eingangs beschriebenen Art zu schaffen, dessen strukturierte Oberflächen genau eine gewünschte, für das Osseointegrationsverhalten optimale Porosität aufweisen, u.zw. mit möglichst großer Präzision, so daß die Stabilität und Belastbarkeit der Oberfläche in einem günstigen Verhältnis zum Volumen der vorgesehenen Struktur der Oberfläche steht.

Diese Aufgabe wird erfindungsgemäß dadurch gelöst, daß die strukturierte Oberfläche aus vollem Material gebildet ist und mit einer Vielzahl zylindrischer Vertiefungen, insbesondere Bohrungen, mit Durchmessern von 0,3 bis 1 mm, vorzugsweise 0,5 bis 1 mm, und mit Tiefen zwischen 0,3 und 1 mm, vorzugsweise 0,5 und 1 mm, versehen ist, wobei eine Porosität zwischen 50 und 80 %, vorzugsweise 60 und 70 %, bewirkt ist.

Wesentlich für ein besonders gutes Ossointegrationsverhalten ist weiters, daß die Tiefe der Vertiefungen gleich ist dem Durchmesser derselben.

Gemäß einer bevorzugten Ausführungsform sind zusätzlich zu den zylindrischen Vertiefungen zylindrische Erhebungen mit Durchmessern zwischen 0,3 und 1 mm, vorzugsweise 0,5 und 1 mm, vorhanden, wobei das Verhältnis der Anzahl der zylindrischen Erhebungen zur Anzahl der zylindrischen Vertiefungen 3 : 7 beträgt, und wobei ebenfalls eine Porosität zwischen 50 und 80 %, vorzugsweise 60 und 70 % vorhanden ist.

Bevorzugt sind die zylindrischen Vertiefungen sowie gegebenenfalls die zylindrischen Erhebungen an ebenen knochenseitigen Flächen des Implantats vorgesehen und an den nicht-planen knochenseitigen Flächen kraterförmig strukturierte Vertiefungen mit Tiefen bis zu 0,5 mm vorgesehen.

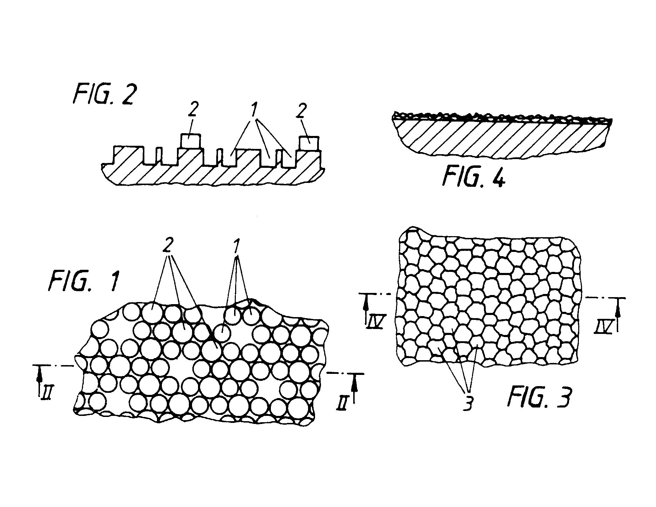

Die Erfindung ist nachstehend anhand eines Ausführungsbeispiels näher erläutert, wobei Fig. 1 eine Draufsicht auf eine ebene, mit dem Knochen in Kontakt gelangende Fläche eines Implantats veranschaulicht. Fig. 2 ist eine Schnittdarstellung eines nach der Linie II-II der Fig. 1 geführten Schnittes. Fig. 3 ist eine Draufsicht auf eine nicht-ebene Fläche eines Implantats und Fig. 4 eine Schnittdarstellung analog zu Fig. 2 nach der Linie IV-IV der Fig. 3. Die in die Fig. 1 und 2 dargestellte strukturierte Oberfläche ist beispielsweise an einem Gelenk-Prothesenimplantat eines Kniegelenks vorgesehen, u.zw. an einer ebenen Fläche einer am Tibiakopf aufliegenden Platte des Gelenk-Prothesenimplantats. Die Strukturierung wird durch eine Vielzahl zylindrischer Vertiefungen 1 mit Durchmessern von 0,3 bis 1 mm, vorzugsweise 0,5 bis 1 mm, und mit Tiefen zwischen 0,3 und 1 mm, vorzugsweise 0,5 und 1 mm erreicht. Wie aus der Zeichnung zu ersehen ist, ist die Strukturierung aus Vollmaterial gebildet, wobei sich das Vollmaterial mindestens bis über die größte Tiefe der Vertiefungen 1 erstreckt. Dies heißt, daß das Implantat auch mit einer Schicht versehen sein kann, die die Vertiefungen 1 aufweist, wobei jedoch die Schicht über ihre Dicke einstückig gestaltet sein muß.

Weiters sind vorteilhaft, jedoch nicht unbedingt notwendig noch zylindrische Erhebungen 2 vorhanden, u.zw. mit Durchmessem zwischen 0,3 und 1 mm, vorzugsweise 0,5 und 1 mm, und Höhen zwischen 0,3 und 1 mm, vorzugsweise 0,5 und 1 mm, wobei das Verhältnis der Anzahl der zylindrischen Erhebungen 2 zur Anzahl der zylindrischen Bohrungen 1 3 : 7 beträgt. Insgesamt wird hierdurch eine Porosität zwischen 50 und 80, vorzugsweise 60 und 70 %, bewirkt, die, falls keine Erhebungen 2 vorhanden sind, alleine von den Vertiefungen 1 erreicht werden muß. Als Porosität ist der Prozentsatz des Luftvolumens über einem Oberflächenbereich, der mit Vertiefungen 1 bzw. Erhebungen 2 versehen ist, gegenüber dem massiven Volumen dieses Oberflächenbereiches zu verstehen.

An den nicht-planen Flächen, mit denen das Implantat 4 mit dem Knochen in Berührung steht, sind kraterförmige Vertiefungen 3 mit Tiefen bis zu 0,5 mm vorgesehen, wie dies in den Fig. 3 und 4 dargestellt ist.

Das Implantat mit der strukturierten Oberfläche ist aus Metall gebildet; insbesondere können hier vorzugsweise folgende Metalle zum Einsatz gelangen: Rein-Titan, eine Titan-Guß- oder Titan-Schmiede-Legierung oder eine Kobalt-Chrom-Legierung.

Dadurch, daß für die strukturierte Oberfläche Erhebungen 2 und Vertiefungen 1 von genau festgelegter Struktur vorgesehen sind, wobei die Vertiefungen 1 und Erhebungen 2 exakt und einfach herstellbar sind, läßt sich die Porosität genau vorherbestimmen, d.h. es können strukturierte Oberflächen je nach gewünschter Porosität in einfacher Weise hergestellt werden, ohne daß man von Zufälligkeiten beim Herstellungsprozeß abhängig ist, wie dies beim Stand der Technik der Fall ist. Hierdurch gelingt es, für Implantate jeweils ein optimales Osseointegrationsverhalten sicherzustellen. Erfindungsgemäß läßt sich eine sehr hohe Porosität erzielen, ohne daß die Gefahr besteht, daß die Porosität beim Manipulieren des Implantates durch Beschädigung an den Erhebungen und Vertiefungen geringer wird. Dies wird durch die erfindungsgemäße zylindrische Gestaltung der Erhebungen 2 und Vertiefungen 1 erreicht.

## Patentansprüche

1. Implantat, insbesondere Gelenk-Prothesenimplantat, mit knochenseitigen strukturierten Oberflächen, dadurch gekennzeichnet, daß die strukturierte Oberfläche aus vollem Material gebildet ist und mit einer Vielzahl zylindrischer Vertiefungen (1), insbesondere Bohrungen, mit Durchmessern von 0,3 bis 1 mm, vorzugsweise 0,5 bis 1 mm, und mit Tiefen zwischen 0,3 und 1 mm, vorzugsweise 0,5 und 1 mm, versehen ist, wobei eine Porosität zwischen 50 und 80 %, vorzugsweise 60 und 70 %, bewirkt ist.

2. Implantat nach Anspruch 1, dadurch gekennzeichnet, daß die Tiefe der Vertiefungen (1) gleich ist dem Durchmesser derselben.

3. Implantat nach Anspruch 1 oder 2, dadurch gekennzeichnet, daß zusätzlich zu den zylindrischen Vertiefungen (1) zylindrische Erhebungen (2) vorhanden sind mit Durchmessern zwischen 0,3 und 1 mm, vorzugsweise 0,5 und 1 mm, wobei das Verhältnis der Anzahl der zylindrischen Erhebungen (2) zur Anzahl der zylindrischen Vertiefungen (1) 3:7 beträgt, und wobei ebenfalls eine Porosität zwischen 50 und 80 %, vorzugsweise 60 und 70 % vorhanden ist.

4. Implantat nach Anspruch 1, 2 oder 3, dadurch gekennzeichnet, daß die zylindrischen Vertiefungen (1) sowie gegebenenfalls die zylindrischen Erhebungen (2) an ebenen knochenseitigen Flächen des Implantats vorgesehen sind, und daß an den nicht-planen knochenseitigen Flächen kraterförmig strukturierte Vertiefungen mit Tiefen bis zu 0,5 mm vorgesehen sind.
